# EUROPÄISCHE PATENTSCHRIFT

(11) **EP 0 905 254 B1**
(45) Veröffentlichungstag und Bekanntmachung des Hinweises auf die Patenterteilung: **26.04.2006**
(21) Anmeldenummer: 98117722.3
(22) Anmeldetag: 18.09.1998
(51) Int. Cl.: C08G 73/02, C12N 15/88

(54) **Verfahren zur Herstellung eines niedermolekularen Polyethylenimins**
Production of a low molecular weight polyethyleneimine
Procédé d'obtention d'un polyéthylèneimines à poids moléculaire bas

(30) Priorität: 30.09.1997 DE 19743135
(43) Veröffentlichungstag der Anmeldung: 31.03.1999
(73) Patentinhaber: Sanofi-Aventis Deutschland GmbH, 65929 Frankfurt am Main (DE)
(72) Erfinder: Kissel, Thomas Prof.Dr., 35037 Marburg (DE); Fischer, Dagmar Dr., 35037 Marburg (DE); Elsässer, Hans-Peter Dr., 35043 Marburg (DE); Bieber, Thorsten, 36272 Niederaula (DE)

(56) Entgegenhaltungen:
- WO-A-96/02655
- DE-C- 665 791
- ZANTA M -A ET AL: "IN VITRO GENE DELIVERY TO HEPATOCYTES WITH GALACTOSYLATED POLYETHYLENIMINE" BIOCONJUGATE CHEMISTRY,AMERICAN CHEMICAL SOCIETY, WASHINGTON,US, Bd. 8, Nr. 6, 1997, Seiten 839-844, XP002920308 ISSN: 1043-1802
- "Polymérisation des imines" 1970 , HERMANN , PARIS XP002178694 399179 * Seite 229 - Seite 230 *
- DR. OTTO-ALBRECHT NEUMÜLLER: "Römpps Chemie-Lexikon, Bd. 5" 1987 , FRANKSCHE VERLAGSHANDLUNG , STUTTGART XP002171107 ISBN: 3-440-04515-3 * Seite 3291 *
- "Encyclopedia of Polymer Science and Engineering" 1985 , JOHN WILEY & SONS , NEW YORK XP002171108 ISBN: 0-471-89540-7 * Seite 695-696 *

## Beschreibung

Die Erfindung betrifft die Herstellung eines niedermolekularen Polyethylenimins.

Ein wesentlicher Therapieerfolg bei der therapeutischen Applikation von DNA *in vivo* hat sich in klinischen Studien am Menschen bislang noch nicht eingestellt. Die Ursachen sind vor allem in der geringen Effizienz des Gentransfers, der begrenzten Expression der genetischen Information [Cotton et al., Meth. Enzymol. 217: 618-644 (1993)] und der mangelnden Biokompatibilität [Choksakulnimitr et al., J. Control. Rel. 34: 233-241 (1995)] der verwendeten kationischen Trägermaterialien zu suchen. Obwohl virale Vektoren, wie Retroviren [Miller, Nature 357: 455-460 (1992)] oder Adenoviren [Mulligan, Science 260: 926-932 (1993)] *in vitro* sehr erfolgversprechende Ergebnisse lieferten, war ihre Anwendung *in vivo* insbesondere aufgrund inflammatorischer und immunogener Eigenschaften sowie der Gefahr der Mutagenese und Integration ins zelleigene Genom limitiert [Crystal, Science 270: 404-410 (1995)]. Als mögliche Alternative boten sich nicht-virale Vektoren an, die nicht nur einfacher zu handhaben sind als virale Systeme, sondern auch sicher und effizient DNA in Zellen einschleusen können [Tomlinson und Rolland, J. Contr. Rel. 39: 357-372 (1996)].

Synthetische Vektoren, basierend auf wasserlöslichen, kationischen Polymeren wie Poly-L-Lysin (PLL) [Wu und Wu, Biotherapy 3: 87-95 (1991)], DEAE-Dextran [Gopal, Mol. Cell. Biol. 5: 1183-93 (1985)]. Dendrimeren [Haensler und Szoka, Bioconjugate Chem. 4: 372-379 (1993)] oder kationischen Methacrylsäure-Derivaten [Wolfert et al., Hum. Gene Ther. 7: 2123-2133 (1996)] haben sich im Laufe der Zeit als Alternative zu der klassische Form der Transfektion, die "Lipofektion" mit kationischen Lipiden [Gao und Huang, Gene therapy 2: 710-722 (1995)] und Amphiphilen [Behr, Bioconjugate Chem. 5: 382-389 (1994)] entwickelt. Der entscheidende Vorteil der "Polyfektion" mit kationischen Polymeren besteht in der endlosen Vielzahl struktureller Variationsmöglichkeiten, die die physikochemischen und biologischen Eigenschaften der Polymere und ihrer Plasmid-Polymer-Komplexe in gewünschter Weise beeinflussen können. Durch die zusätzliche Kopplung zellspezifischer Liganden, wie Transferrin [Wagner et al., Proc. Natl. Acad. Sci. 87: 3410-3414 (1990)], Asialoglykoprotein [Wu und Wu, J. Biol. Chem. 262: 4429-4432 (1987)], sowie verschiedenen Antikörpern [Trubetskoy et al., Bioconjugate Chem. 3: 323-327 (1992)] und Carbohydraten [Midoux et al., Nucleic Acid Research 21: 871-878 (1993)] ließ sich die Effizienz dieser Vektoren erheblich steigern.

Polyethylenimin (PEI), ein kationisches Polymer mit dreidimensionaler, verzweigter Struktur, hat in einer Vielzahl verschiedener adhärenter und Suspensions-Zellinien zu teilweise überdurchschnittlich hohen Transfektionsraten geführt [Boussif et al., Gene Therapy 3: 1074-1080 (1996)]. 3T3 Fibroblasten beispielsweise konnten *in vitro* zu 95% transformiert werden. Der PEI-vermittelte Gentransfer *in vivo* am Gehirn von Mäusen bewirkte eine Langzeit-Expression von Reportergenen und dem Bcl2-Gen in Neuronen und Gliazellen, die in der gleichen Größenordnung liegt wie beim adenoviralen Gentransfer [Abdallah et al., Hum. Gene Ther. 7: 1947-1954 (1996)].

Polyethylenimin verfügt über herausragende Eigenschaften im Vergleich zu anderen literaturbekannten Polykationen wie PLL [Zenke et al., Proc. Natl. Acad. Sci. 87: 3655-3659 (1990)], Methacrylat-Derivaten [Cherng et al., Pharm. Res. 13: 1038-1042 (1996)] oder DEAE-Dextran [Gopal, Mol. Cell. Biol. 5: 1183-93 (1985)]. Aufgrund seiner quervernetzten Struktur und hohen Ladungsdichte ist es in der Lage, Plasmide in hohem Maße zu kondensieren und komplexieren. In Form solcher Komplexe kann DNA dann in Zellen eingeschleust werden. Die Mechanismen der Aufnahme, der intrazellulären Prozessierung und der lysotropen Aktivität der PEI-Plasmid-Komplexe sind bis heute nicht endgültig geklärt.

Der entscheidende Vorteil des PEI scheint eine pH-abhängige Veränderung seiner Struktur zu sein, die zu einer Destabilisierung endosomal-lysosomaler Kompartimente führt und damit die Freisetzung der Komplexe in das Zytoplasma erleichtert. Insbesondere die Aminofunktionen mit unterschiedlichen pKa-Werten des Moleküls sollen für eine ausgeprägte Pufferkapazität des PEI verantwortlich sein ("proton sponge"), die bei der Azidifizierung der Endosomen zu einer Protonierung der Polymere unter Quellung und damit zur Ruptur der Vesikelmembranen führt. Der durch die endosomale ATPase vermittelte Einstrom von Protonen bedingt vermutlich gleichzeitig den passiven Influx anionischer Chloride, der in Anwesenheit von PEI zu einer massiven Erhöhung der Gesamtionenkonzentration und damit zur osmotischen Schwellung der Endosomen führt [Behr, Chimia 51: 34-36 (1997)]. Lysosomotrope Agentien wie Chloroquin, die z.B. für die Transfektion von PLL essentiell sind, haben daher keinen Einfluß auf die Transfektionsrate der PEIs [Remy und Behr, J. Lip. Res. 6: 535-544 (1996)].

In WO 9602655 A1 wurde die Verwendung von hochmolekularem Polyethylenimin mit einem Molekulargewicht von 50 kDa und 800 kDa (Molmasse 50.000 g/mol bzw. 800.000 g/mol) für die Transfektion von DNA in Zellen beschrieben.

Kommerziell erhältliches PEI weist nach Angabe.des Herstellers (z.B. Fluka, Neu Ulm) ein Molekulargewicht von 600 -1000 kDa auf. Derartige PEI-Präparationen mit hohem Molekulargewicht ("high molecular weight PEI": HMW-PEI) besitzen bereits ab einer Konzentration von 0,01 mg/ml und nach kurzer Inkubation von 3 h eine deutliche Zytotoxizität. Des weiteren ist die Polyethyleniminstruktur weder enzymatisch noch hydrolytisch zu spalten und demnach biologisch nicht abbaubar. HMW-PEI ist zudem vermutlich weder über Faeces noch über die Niere ausscheidbar.

In Folge dessen ist die in vivo Verabreichung des bislang verwendeten HMW-PEI zum Beispiel im Rahmen der Gentherapie mit erheblichen Risiken belastet.

Gegenstand der Erfindung ist ein Verfahren zur Herstellung eines Polyethylenimins mit einem Molekulargewicht unter 50.000 Da, vorzugsweise zwischen 500 Da und 30.000 Da ("Low molecular weigth PEI": LMW-PEI).

Vorzugsweise weist das LMW-PEI ein Molekulargewicht von 500 bis 30.000 Da auf. In einer bevorzugten Ausführungsform der Erfindung weist das LMW-PEI ein Molekulargewicht von 1.000 bis 5.000 Da auf. Besonders bevorzugt ist ein Molekulargewicht von etwa 2.000 Da.

Gegenstand der Erfindung ist ein Verfahren zur Herstellung von niedrigmolekularem, kationischem Polymerkonjugat (LMW-PEI) mit einem Molekulargewicht von unter 50.000 Da auf der Basis von Polyethylenimin (PEI) durch ringöffnende Polymerisation von Aziridin (monomeres Ethylenimin), wobei monomeres Ethylenimin in wässriger Lösung durch Zusatz von Salzsäure polymerisiert wird, die wässrige Lösung 0,1 %ig bis 90 %ig an monomerem Erhylenimin und 0,1 %ig bis 10 %ig an konzentrierter Salzsäure ist, die Polymerisation bei einer Reaktionstemperatur von 30°C bis 70°C durchgeführt wird und die Reaktionszeit 1 bis 30 Tage beträgt.

Vorzugsweise erfolgt dabei die Herstellung des Ethylenimins aus Ethanolamin nach der Methode von Wenker (JACS 57: 2328 (1935)). Der Siedepunkt liegt vorzugsweise bei 55,0 - 56,0°C.

In der Patentanmeldung Ger Pat. 665,791 (1938) ist die Synthese von PEI durch Zugabe von Katalysatoren, wie Säuren oder Bortrifluorid zum flüssigen monomeren Ethylenimin beschrieben. Entsprechend der Erfindung wird in Analogie zu Dick et al., J.Macromol. Sci. A4: 1301-1314 (1970) monomeres Ethylenimin in wässriger Lösung unter Zusatz von Salzsäure polymerisiert.

Für die Polymerisation wird eine 0,1 %ige bis 90%ige Ethylenimin (Monomer) Lösung in destilliertem Wasser unter Rühren hergestellt und 0,1% bis 10% konzentrierte Salzsäure (37%) als Katalysator zugefügt. Die Polymerisation erfolgt über 1-30 Tage, vorzugsweise 4 Tage bei einer Temperatur von 30-70°C, vorzugsweise 50°C.

Die Charakterisierung der Polymere erfolgt z.B. mit 13C-NMR Spektroskopie, Sizeexclusion Chromatographie, Lichtstreuung und/oder Viskosimetrie. Das Verfahren zur Bestimmung des Molekulargewichts mit Hilfe der Lichtstreuungsmethode ist grundsätzlich in B. Vollmert (1962) "Grundriss der Makromolekularen Chemie", Springer Verlag, Berlin, Seiten 216-225 beschrieben. Vorzugsweise wird die Molekulargewichtsbestimmung mittels der Lichtstreuungsmethode, insbesondere der Laserstreulichtmessung, z.B. unter Verwendung eines Lichtstreuphotometers, z.B. Lichtstreuphotometer Wyatt Dawn DSP bei 633 nm nach Direktinjektion in eine K5-Meßzelle durchgeführt. Das Molekulargewicht kann beispielsweise mit Hilfe von in Toluol bestimmten Kalibrierkonstanten und der bekannten Probeneinwaage bestimmt werden.

Mit dem beschriebenen Verfahren kann niedrigmolekulares (niedermolekulares) PEI (LMW-PEI) mit Molekülgrößen zwischen 500 Da und 50.000 Da hergestellt werden. Das Molekulargewicht des niedrigmolekularen PEI (LMW-PEI) liegt damit deutlich unter dem des HMW-PEI und deutlich unterhalb der Nierenschwelle von 50 kDa, so daß eine renale Elimination gewährleistet sein sollte.

Überraschend stellte sich heraus, daß LMW-PEI in Bezug auf Wirksamkeit als Vektor für die Einführung von Nukleinsäuren bzw. Nukleinsäurekonstrukten in Zellen und in seiner biologischen Verträglichkeit dem HMW-PEI deutlich überlegen ist. Am besten geeignet erwies sich LMW-PEI mit Molekülgrößen zwischen 1000 Da und 30.000 Das LMW-PEI ist in der Lage, DNA zu binden, zu kondensieren und zu positivieren. LMW-PEI mit einem Molekulargewicht von beispielsweise etwa 2000 Da führte im Komplex mit DNA enthaltend ein Reportergen [in Gegenwart von Serum] zu 100fach höheren Reportergenexpressionen in Säugerzellen [beispielsweise in Mausfibroblasten (3T3) und humanen endothelialen Zellen (ECV 304)], als das kommerzielle hochmolekulare (HMW) PEI. Gleichzeitig war die Zytotoxizität des LMW-PEI auf Fibroblasten im Vergleich zum HMW-PEI deutlich reduziert.

Das LMW-PEI bzw. ein Vektor, der das LMW-PEI enthält, kann beispielsweise zur Einführung einer Nukleinsäure in eine Zelle/Zielzelle, wobei die Zelle/Zielzelle Endothelzelle, einen Lymphozyt, einen Makrophagen, eine Leberzelle, einen Fibroblast, eine Muskelzelle oder eine Epithelzelle ist, verwendet werden.

Da das efindungsgemäße LMW-PEI weniger stark verzweigt ist als HMW-PEI und daher mehr Aminogruppen enthält als HMW-PEI, bietet LMW-PEI in weitaus besserem Maße als HMW-PEI die Möglichkeit der Kopplung an einen zellspezifischen Liganden. Gegenstand der Erfindung ist somit die Kopplung des LMW-PEI an einen zellspezifischen Liganden und die Verwendung des Kopplungsproduktes im Komplex mit einer viralen oder nicht viralen Nukleotidsequenz für die Einführung der Nukleotidsequenz in eine Zelle oder für die Verabreichung des Komplexes an einen Säuger zur Prophylaxe oder Therapie einer Erkrankung. Die Möglichkeiten der Herstellung und Kopplung von zellspezifischen Liganden ist bereits in den Patentanmeldungen EP97101506.0 und DE19649645.4 ausführlich beschrieben worden. Auf diese Patentanmeldungen wird ausdrücklich Bezug genommen.

### Beispiele:

### 1) Methoden

### a) Herstellung von niedrigmolekularem Polyethylenimin (LMW-PEI)

LMW-PEI wird aus Aziridin durch eine ringöffnende Polymerisation in wäßriger Lösung unter Säurekatalyse gewonnen. Dazu wurde beispielsweise eine 10%ige Ethyleniminmonomer-Lösung in Wasser (5 ml Ethyleniminmonomer + 45 ml destilliertes Wasser, Auflösung unter Rühren) unter Zusatz von. 1% (0,5 ml) konzentrierter Salzsäure (37 %) als Katalysator 4 Tage lang bei 50°C gerührt, einrotiert und unter Vakuum bei Raumtemperatur getrocknet. Die Molekulargewichtsbestimmungen wurden mittels Laserstreulichtmessung (Lichtstreuphotometer Wyatt Dawn DSP) bei 633 nm nach Direktinjektion in eine K5-Meßzelle durchgeführt. Die Molmassen werden aufgrund der in Toluol bestimmten Kalibrierkonstanten und der bekannten Probeneinwaage bestimmt.

Die Molekulargewichtsbestimmung mit Hilfe der Lichtstreuanalyse ergab 2000 Da. Vergleichsweise hatte das kommerziell (Fa. Fluka, Neu Ulm) erhaltene PEI ein Molekulargewicht entsprechend der Lichtstreuanalyse von 791 kDa (HMW-PEI).

Beide Präparate (LMW-PEI und HMW-PEI) wurden vergleichsweise geprüft.

### b) Präparation der Polynukleotidkomplexe

Die Komplexierung der Plasmid-DNA mit den PEIs erfolgt in Anlehnung an die Methode von Boussif et al. [Boussif et al., Proc. Natl. Acad. Sci. 92: 7297-7301 (1995)]. 9 mg der 50%igen kommerziellen HMW-PEI-Lösung bzw. 9 mg LMW-PEI wurden in 9 ml bidestilliertem Wasser gelöst, mit 1 N HCl auf pH 7,4 eingestellt und mit Wasser auf ein Endvolumen von 10,0 ml aufgefüllt. Die fertigen Lösungen wurden sterilfiltriert (0,2 µm) und konnten bei 4°C längere Zeit gelagert werden.

Zur Komplexbildung wurden 10 µg Plasmid und die verschiedenen Mengen der PEI-Stammlösungen in jeweils 150 mM NaCl auf ein Endvolumen von 250 µl verdünnt und im Vortex gemischt. Einen Überblick über die Ansatz- und Äquivalentverhältnisse der Komplexe gibt die Tabelle 1. Nach 10 minütiger Inkubation bei Raumtemperatur wurden die Polymerlösungen portionsweise zu den Plasmidlösungen getropft und im Vortex gemischt. Bevor die Komplexe dem Zellkulturmedium zugesetzt wurden, wurden sie noch einmal 10 min lang inkubiert.

### c) Agarose Shiftassay

Die Plasmid-Bindungskapazität der verschiedenen PEls wurde in Agarosegel-Shiftassays kontrolliert. Dazu wurden 1,35-27 µg HMW-PEI und 2,7-90 µg LMW-PEI mit jeweils 10 µg Plasmid komplexiert-(Tab. 1). 50 µl Aliquots wurden auf ein ca. 0,5 cm dickes Gel aus 1% (w/v) Agarose aufgetragen und in Tris-EDTA-Puffer pH 7,4 bei 80 mV 2 h lang entwickelt. Die Lokalisation, der DNA wurde bei 254 nm nach Reaktion mit Ethidiumbromid visualisiert.

Zur Verdrängung der Plasmide aus den Komplexen wurden zu jeweils 10 µg-DNA-Komplex 50 bzw. 100 µl einer Dextransulfatlösung (Mw 5000, 10 mg/ml, Sigma, Deisenhofen) 30 min nach der Komplexformation zugesetzt.

### d) Zellkulturen

### L929 Mausfibroblasten wurden unter den dem Fachmann geläufigen

Standardbedingungen kultiviert. Diese Zellen wurden in einer Dichte von 8000 Zellen/Well in 96-Well-Zellkulturschalen ausgesät und 24h lang kultiviert, bevor sie für Toxizitätsexperimente verwendet wurden.

Die Kultivierung von 3T3 Fibroblasten erfolgte gleichermaßen unter Standardbedingungen.

ECV 304 (ATCC, Rockville, MD USA), eine spontan transformierte, adhärente, humane Endothelzellinie, die aus einer scheinbar normalen Corda Umbilicalis etabliert wurde, wurde in Dulbecco's modified Eagle's medium (DMEM) (Gibco, Eggenstein) mit 5% fötalem Kälberserum (FKS), 5% Pferdeserum und 1% N-Acetyl-L-alanyl-L-glutamin (alle Gibco, Eggenstein) kultiviert.

Die bei 37°C, 95% relativer Luftfeuchtigkeit und 5% CO2 inkubierten Zellen wurden zweimal pro Woche nach Erreichen der Konfluenz mit Trypsin/EGTA-Lösung (2,5% Trypsin-Stocklösung, 50 mM Ethylenglycoltetraessigsäurelösung, PBS pH 7,4 im Verhältnis 1:1:8) passagiert. Da sich die Zellen nicht einzeln vom Untergrund ablösten, sondern Zellcluster bildeten, wurde jeweils eine 1/8 Passage durchgeführt.

Zerebrale kapilläre Endothelzellen wurden nach der Methode von Bowman et al. ((1983) Ann. Neurol. 14: 396-402) und Mischek et al. [Mischek et al., Cell. Tiss. Res. 256: 221-226 (1989)] isoliert und kultiviert. Für Transfektionsversuche wurden sie unmittelbar nach der Isolierung in 6-Well-Zellkulturschalen ausgesät und bis zu ca. 50% Konfluenz kultiviert.

### e) Zytotoxizitätsstudie

Die Toxizität der Polymere wurde mit dem MTT-Assay nach der Methode von Mosmann et al. [Mosmann, J. Immunol. Methods 65: 55-63 (1983)] an L929 Mausfibroblasten bestimmt. Die Verdünnungsreihen der Polymere wurden in DMEM mit 10% FKS und 2 mM Glutamin hergestellt und sterilfiltriert (0,2 µm, Schleicher & Schuell, Dassel). Falls erforderlich, wurden pH-Wert und Osmolarität der Lösungen korrigiert. Nach einer Vorinkubation von 24 h wurden die Zellen mit den Polymerlösungen versetzt und 1, 3, 12 und 24 h inkubiert. Die Viabilität der Zellen wurde UVphotometrisch durch Messung der Formazankonzentration quantifiziert.

In einer zweiten Serie von Experimenten wurden die Zellen generell nur 1 h lang mit den Polymeren behandelt, gewaschen und in PEI-freiem Zellkulturmedium bis zu 3, 12 und 24 h weiterkultiviert. Die Auswertung erfolgte wie oben beschrieben.

### f) Transfektionen

Die in 3 cm²-Petrischalen ausgesäten 3T3 Mausfibroblasten und ECV 304 Zellen sowie die in 6-Well-Kulturschalen ausgesäten primären Endothelzellen, wurden unmittelbar vor den Experimenten mit PBS pH 7,4 gewaschen und mit neuem Medium supplementiert mit Serum versorgt. Die Komplexe des HMW-PEI und des LMW-PEI entsprechend 3,33 µg DNA pro Well oder Schale wurden zugesetzt und 1 h lang bei 37°C inkubiert. Die Zellen wurden 60 Stunden lang nachinkubiert und die Luciferase- bzw. β-Galactosidaseaktivität bestimmt analog den Angaben des Herstellers. [Komplexe des HMW-PEIs bzw. LMW-PEIs sind die entsprechenden Vektoren; sie enthalten HMW-PEI bzw. LMW-PEI und Nukleinsäuren, in diesem Fall Plasmid-DNA)],

### Beispiel 2: Ergebnisse

### a) physikochemische Eigenschaften der PEIs

Das Verhalten der Polymere im Hinblick auf ihre Reaktion im endosomal-lysosomalen Kompartiment wurde durch Quellungsstudien bei verschiedenen pH-Werten im Bereich 4-10 in 0,1 M Phosphatpuffer bestimmt. Während sich HMW-PEI klar und ohne Rückstand bei pH 9 und 10 löste, war ab pH 8 eine intensive Trübung zu beobachten: Diese Trübung war bei pH 7 und 8 weitgehend stabil. Sedimentationserscheinungen traten erst nach mehreren Stunden auf. Bei pH-Werten im aciden Bereich hingegen, kam es innerhalb von 30 min zur Bildung eines Sediments, das leicht resuspendiert werden konnte. LMW-PEI zeigte unter den gleichen Bedingungen keine Trübung oder Präzipitätion, sondern ging klar in Lösung.

### b) Zytotoxizitätsstudien

Die Toxizität der PEls wurde *in vitro* an L929 Mausfibroblasten bestimmt, welche von verschiedenen Standardorganisationen als Standardzellkulturmodell zur Bestimmung der Zytotoxizität und Biokompatibilität von Polymeren empfohlen werden. In vorangegangenen Experimenten wurde eine direkte, lineare Proportionalität zwischen der gemessenen Absorption des gebildeten Formazans und der Zellzahl im Bereich 1x10³ und 3x10⁴ Zellen festgestellt. 8000 Zellen/Well wurden nach einer 24 stündigen Wachstumsphase mit den Polymerlösungen versetzt und 1, 3, 12 und 24 h lang inkubiert. Die beobachteten toxischen Effekte des HMW-PEI und des LMW-PEI waren im Bereich von 0-1,0 mg/ml über einen Zeitraum von bis zu 24 h zeit- und konzentrationsabhängig, wobei die Zytotoxizitätsprofile für hoch- und niedermolekulares PEI deutliche Unterschiede zeigten. So lag die IC50 bei HMW-PEI zwischen 0,06 mg/ml (1 h Inkubation) und 0,04 mg/ml (24 h Inkubation) während bei LMW-PEI Konzentrationen zwischen 0,1 und 1,0 mg/ml erst nach 12 Stunden Inkubation.toxisch wurden, eine IC50 erst nach 24 h Inkubationsdauer ermittelbar war und etwa bei 0,1 mg/ml lag.

### c) Agarosegel-Shiftassay

Um das optimale Bindungs- und Mengenverhältnis zwischen Plasmid und PEI zu ermitteln, wurde eine konstante Menge Plasmid (10 µg) mit verschiedenen Konzentrationen HMW-PEI und LMW-PEI nach Vorschrift komplexiert und elektrophoretisch analysiert. Tabelle 1 gibt eine Übersicht über das Ansatzverhältnis der untersuchten Komplexe, das Volumen an verwendeter Stammlösung und die absolute PEI-Menge.

**Tab. 1: Übersicht über die Ansatzverhältnisse der zur Elektrophorese und Transfektion verwendeten Komplexe mit a) HMW-PEI und b) LMW-PEI**

| a) | | |
|---|---|---|
| Ansatzverhältnis Plasmid/HMW-PEI | Volumen der Stammlösung | HMW-PEI absolute Menge |
| [Äquivalente] | [µl] | [µg] |
| 1+1 | 3 | 1,35 |
| 1+6,67 | 20 | 9 |
| 1+10 | 30 | 13,5 |
| 1+13,33 | 40 | 18 |
| 1+20 | 60 | 27 |

| b) | | |
|---|---|---|
| Ansatzverhältnis Plasmid/LMW-PEI | Volumen der Stammlösung | LMW-PEI absolute Menge |
| [Äquivalente] | [µl] | [µg] |
| 1+2 | 3 | 2, 7 |
| 1+13,33 | 20 | 18 |
| 1+20 | 30 | 27 |
| 1+26,67 | 40 | 36 |
| 1+40 | 60 | 54 |
| 1+53,33 | 80 | 72 |
| 1+66,66 | 100 | 90 |

Die Lokalisation der Plasmide und ihrer Komplexe wurde mittels Ethidiumbromid-Färbung sichtbar gemacht. Die DNA bildete zwei fluoreszierende Banden, die der supercoiled und der zirkulären Form des Plasmids entsprechen und die in Richtung Anode wanderten. HMW-PEI und LMW-PEI waren mit Ethidiumbromid nicht detektierbar.

Die Komplexierung von DNA mit HMW-PEI im Verhältnis 1+1 führte zu einer partiellen, noch unvoflständigen Retardierung des Plasmids am Auftragsort. Die geringere Gesamtladung und/oder ein größerer Durchmesser hinderten den entstandenen Komplex an der Wanderung in der Gelmatrix.

Komplexe im Verhältnis 1+6 bis 1+20 waren nicht detektierbar, da sie keine Fluoreszenz zeigten, ein Hinweis auf den Ethidiumbromidausschluß der Plasmide bedingt durch eine effiziente Kondensation und physikalische Kompression ihrer Struktur durch das HMW-PEI. Die Anion/Kation-Verhältnisse liegen hier bei 1:1,2 (1+6) bis 1:4 (1+20). Die Komplexe sollten demzufolge eine positive Gesamtladung besitzen.

2,7 µg LMW-PEI konnten 10 µg Plasmid fast vollständig binden und retardieren. Der Komplex wies jedoch noch eine negative Gesamtladung auf und orientierte sich zur Anode. Die vollständige Kationisierung und Kondensation der DNA ließ sich jedoch erst ab 54 µg LMW-PEI beobachten,

Um den Effekt der Kondensation durch hoch- und niedermolekulares PEI zu verifizieren, wurde die DNA aus den fertigen Komplexen mit einem Überschuß an Dextransulfat verdrängt, das eine Konkurrenzreaktion mit den kationischen Polymeren eingeht. Sowohl im Falle des HMW-PEI als auch des LMW-PEI konnte die DNA aus den Komplexen.wieder freigesetzt werden und ganz oder teilweise in die Gelmatrix wandern. Die Interkalation von Ethidiumbromid war wieder möglich und somit die DNA durch Fluoreszenz detektierbar.

### d) In vitro Transfektionseffizienz

Die Transfektionseffizienz der PEI-Komplexe wurde sowohl mit Zellinien (3T3 Mausfibroblasten und humane endotheliale Zellinie ECV304) als auch mit Primärkulturen (kapilläre Endothelzellen aus Schweinehirn) bestimmt. Als Reportergen wurde der kommerziell erhältliche pGL3-control Vektor der Fa. Promega verwendet, der ein Luciferasegen unter der Kontrolle eines SV 40-Promotors und -Enhancers trägt. Die Ansatzverhältnisse der Komplexe bezüglich Plasmid und Polymer entsprachen denen, die bei der Elektrophorese verwendet wurden.

Geprüft wurden Konzentrationen von 1,35 µg bis 27 µg HMW-PEI/10 µg DNA. Das Maximum der Transfektion zeigte sich bei 18 µg HMW-PEI. Eine weitere Erhöhung der Polymerkonzentration führte nur zu einer relativ geringfügigen Verminderung der Luciferase-Expression.

Beim LMW-PEI wurden Konzentrationen von 20-80 µg LMW-PEI/10 µg DNA geprüft. Im Gegensatz zu HMW-PEI konnte mit steigender Konzentration an LMW-PEI eine stetige Zunahme der Transfektionseffizienz in den ECV-Zellen detektiert werden. Bei 80 µg LMW-PEI/10 µg DNA wurde eine etwa 100fach höhere Reportergenaktivität gemessen als bei Verwendung der maximal wirksamen Dosis von 18 µg HMW-PEI/10 µg DNA. Ein Rückgang der Luciferaseexpression wie beim hochmolekularen PEI war auch bei den höchsten Konzentrationen von LMW-PEI nicht zu beobachten. Die Versuche mit ECV-Zellen und den 3T3 Zellen lieferten identische Ergebnisse.

Zum Vergleich wurden die Transfektionsstudien mit β-Galaktosidase als Reportergen auch an endothelialen Primärzellkulturen mit den maximal tolerablen, nicht zytotoxischen Dosierungen (MTD) von HMW-PEI und LMW-PEI Komplexen durchgeführt. Die MTD in vitro lag bei 13,5 µg HMW-PEI/10 µg DNA und bei 90 µg LMW-PEI/10 µg DNA. Kultivierte kapilläre Endothelzellen aus Schweinehirnen, die mit Komplexen aus 10 µg DNA und 13,5 µg HMW-PEI inkubiert wurden, ließen sich kaum transfizieren. Nur 2-3 Zellen pro Kulturnapf (Well) zeigten die charakteristische Blaufärbung im Bereich der Zellkerne. Die Erfolgsrate der Transfektion lag unter 1% der behandelten Zellen. Die Inkubation mit Komplexen aus 90 µg LMW-PEI und 10 µg DNA hingegen führte zu einer deutlichen Expression des Markerproteins in den Endothelzellen. Der prozentuale Anteil an blau gefärbten Zellen pro Kulturnapf in Relation zur Gesamtzellzahl, d.h. die Erfolgsrate der Transfektion lag zwischen 5% und 10%. In keinem Fall konnten toxische Effekte der Polymer/DNA-Komplexe an den Zellen lichtmikroskopisch beobachtet werden.

## Patentansprüche

1. Verfahren zur Herstellung eines niedermolekularen Polyethylenimins (LMW-PEI) mit einem Molekulargewicht unter 50 000 Da, **dadurch gekennzeichnet, dass** monomeres Ethylenimin in wässriger Lösung durch Zusatz von Salzsäure polymerisiert wird, wobei die wässrige Lösung 0,1 %ig bis 90 %ig an monomerem Ethylenimin und 0,1 %ig bis 10 %ig an konzentrierter Salzsäure ist, die Polymerisation bei einer Reaktionstemperatur von 30°C bis 70°C durchgeführt wird und die Reaktionszeit 1 bis 30 Tage beträgt.

## Revendications

1. Procédé de préparation d'une polyéthylène-imine à faible poids moléculaire (LMW-PEI), présentant un poids moléculaire inférieur à 50 000 Da, **caractérisé en ce que** l'éthylène-imine monomère est polymérisé en solution aqueuse par addition d'acide chlorhydrique, la solution aqueuse étant constituée de 0,1 % à 90 % d'éthylène-imine monomère et de 0,1 % à 10 % d' acide chlorhydrique concentré, la polymérisation étant effectuée à une température de réaction de 30°C à 70°C et la durée de la réaction étant de 1 à 30 jours.

## Claims

1. A process for preparing a low molecular weight polyethylenimine (LMW PEI) having a molecular weight of less than 50,000 Da, which comprises monomeric ethylenimine being polymerized in aqueous solution by adding hydrochloric acid, wherein the aqueous solution is from 0.1% strength to 90% strength with respect to monomeric ethylenimine and from 0.1% strength to 10% strength with respect to concentrated hydrochloric acid, the polymerization is carried out at a reaction temperature of from 30°C to 70°C and the reaction time is from 1 to 30 days.
